# EUROPÄISCHE PATENTANMELDUNG

(11) **EP 1 288 207 A1**
(43) Veröffentlichungstag der Anmeldung: **05.03.2003**
(21) Anmeldenummer: 02018159.0
(22) Anmeldetag: 19.08.2002
(51) Int. Cl.: C07D 277/10

(54) **Verfahren zur Herstellung von 4,5-Dihydro-1,3-thiazolen**

(30) Priorität: 31.08.2001 DE 10142749
(71) Anmelder: Haarmann & Reimer GmbH, 37603 Holzminden (DE)
(72) Erfinder: Joschek, Katrin, Dr., 51061 Köln (DE); Vidal-Ferran, Anton, Dr., 50733 Köln (DE); Jautelat, Manfred, Dr., 51399 Burscheid (DE); Schelhaas, Michael, Dr., 50733 Köln (DE)
(74) Vertreter: Zobel, Manfred, Dr.

(57) **Zusammenfassung**

Die vorliegende Erfindung betrifft ein verbessertes und zugleich ökonomischeres Verfahren zur Synthese von 4,5-Dihydro-1,3-thiazolen der Formel (I), wobei die gesamte Reaktionssequenz als Eintopfsynthese durchgeführt wird.

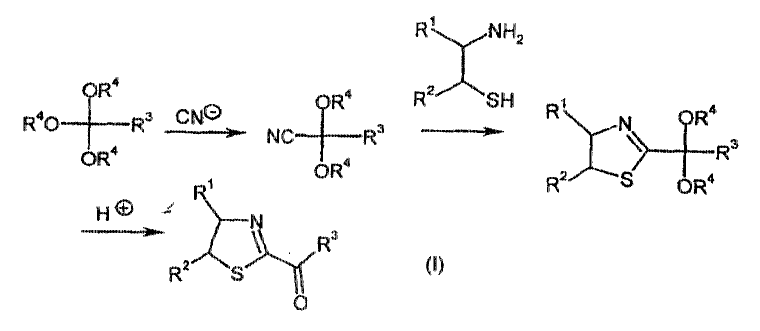

## Beschreibung

Die vorliegende Erfindung betrifft ein verbessertes und zugleich ökonomischeres Verfahren zur Synthese von 4,5-Dihydro-1,3-thiazolen.

4,5-Dihydro-1,3-thiazole sind seit langem bekannte Stoffe, die u.a. als zentrale Intermediate für die Synthese von auf Dihydrothiazol- und Thiazol-basierten Wirkstoffen im Agro- und Pharmabereich Verwendung finden.

Zur Herstellung von 4,5-Dihydro-1,3-thiazolen wird ein effizienter Syntheseweg mit sehr guten Selektivitäten und Ausbeuten benötigt. Die erforderlichen Edukte dazu müssen im technischen Maßstab verfügbar sein.

Die Substanzklasse der 4,5-Dihydro-1,3-thiazole (I) ist bekannt und ihre Synthese wird u.a. in DE-A 1 964 276 und US-A 3,678,064 beschrieben. Nach dem beschriebenen Syntheseweg erhält man ausgehend von 1-Amino-2-alkanthiolen (II) durch Umsetzung mit 2,2-Dialkoxyalkannitrilen (III) die Ketale (IV), die durch Hydrolyse in die gewünschten 4,5-Dihydro-1,3-thiazole (I) umgesetzt werden. Für die Darstellung von 2,2-Dialkoxyalkannitrilen (III) gemäß DE-A 1 964 276 werden inakzeptable 40 Tage Reaktionszeit benötigt. Ein verbessertes Verfahren ist in Synthesis 1983, 498-500 mit 83 Gew.- % Ausbeute beschrieben. Die Reaktionszeit beträgt hier 3 bis 12 Stunden. 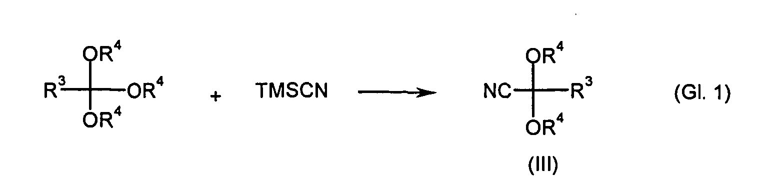 TMSCN = Trimethylsilylcyanid

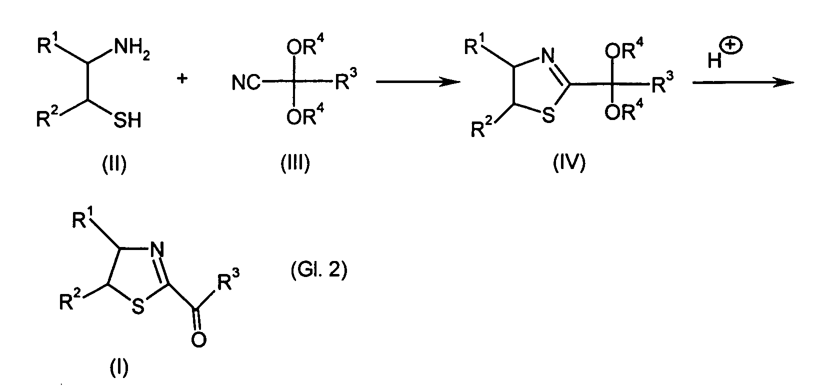

In den Formeln bedeuten R¹, R², R³ und R⁴ - unabhängig voneinander - Wasserstoff oder einen organischen Rest mit 1 bis 10 Kohlenstoffatomen.

Im ersten Schritt in Gleichung 2 werden 1,57 eq. 1-Amino-2-alkanthiol (II) benötigt, das sehr teuer ist. Der Nachteil in der beschriebenen Synthesesequenz liegt in den aufwändigen Aufarbeitungsschritten mit Isolierung der Zwischenstufen. Besonders kritisch im Hinblick auf die technische Anwendung ist die Hydrolyse mit einem Überschuss an konzentrierter Schwefelsäure (15.5eq.), da diese großen Säuremengen anschließend vernichtet werden müssen, was mit starker Exothermie verbunden ist. Außerdem fällt bei der Neutralisation eine große Menge Salz an, was unter ökologischen Aspekten unerwünscht ist. Nach jeder Stufe des beschriebenen Verfahrens erfolgt eine wässrige Aufarbeitung mit anschließender Reinigung. Die wässrige Aufarbeitung geht immer mit einem erheblichen Salzanfall einher, was ebenfalls im technischen Verfahren von Nachteil ist.

Es galt nun das Verfahren dahingehend zu verbessern, dass eine technische Realisierung des Prozesses unter Berücksichtigung ökologischer Aspekte verwirklicht werden kann und die Nachteile des bisherigen Verfahrens umgangen werden. Diese Aufgabe konnte erfindungsgemäß gelöst werden.

In überraschender Weise wurde gefunden, dass die gesamte Synthesesequenz als Eintopfsynthese ohne aufwändige Aufarbeitungsschritte durchgeführt werden kann.

Gegenstand der Erfindung ist daher ein Verfahren zur Herstellung von 4,5-Dihydro-1,3-thiazolen der Formel (I) nach der nachfolgenden Reaktionssequenz 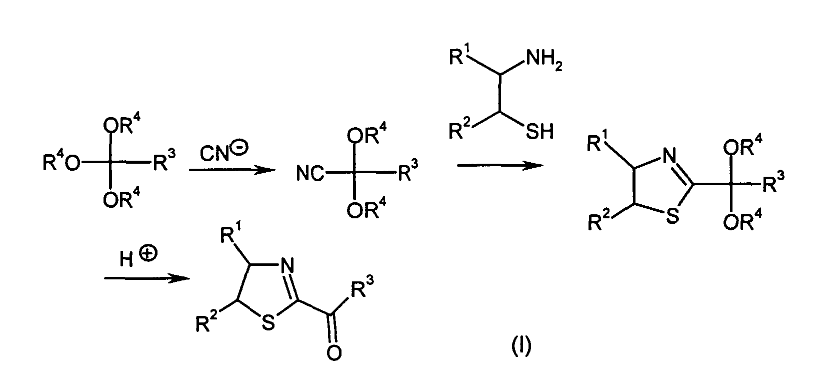 wobei R¹, R², R³ und R⁴ - unabhängig voneinander - Wasserstoff oder einen organischen Rest mit 1 bis 10 Kohlenstoffatomen bedeuten,
dadurch gekennzeichnet, dass die gesamte Reaktionssequenz als Eintopfsynthese ohne Isolierung der Zwischenstufen durchgeführt wird.

Funktionelle Gruppen, mit denen die organischen Reste R¹, R² und R³ substituiert sein können sind z.B. Alkohole und Halogene.

Vorzugsweise bedeuten R¹ und R² Wasserstoff oder Alkylgruppen mit 1 bis 10 Kohlenstoffatomen. Besonders bevorzugt stehen R¹ und R² für Wasserstoff.

Vorzugsweise bedeutet R³ eine Alkylgruppe mit 1 bis 10 Kohlenstoffatomen. Besonders bevorzugt steht R³ für Ethyl.

Vorzugsweise bedeutet R⁴ eine Alkylgruppe mit 1 bis 10 Kohlenstoffatomen. Besonders bevorzugt steht R⁴ für Methyl, Ethyl oder Propyl.

Die Gesamtausbeute liegt hier z. B. für die Synthese des 2-Propionyl-4,5-dihydro-1,3-thiazols (I, R³= Et) bei 40 %. Zusätzlich konnte die Menge an 1-Amino-2-alkanthiol (II) von 1.57 eq. auf 1.1 eq. abgesenkt werden.

**Tabelle:**

| Vergleich der Ausbeuten | | | | | | | |
|---|---|---|---|---|---|---|---|
| Verfahren | Menge (II) [mol] | Menge (III) [mol] | R¹ | R² | R³ | R⁴ | Gesamtausbeute [%] |
| DE-A 1 964 276 | 1,57 | 1,0 | H | H | C₂H₅ | C₂H₅ | 16 |
| Beispiels 3 (Erfindung) | 1,10 | 1,0 | H | H | C₂H₅ | CH₃ | 40 |

Der Vorteil der Erfindung liegt zum einen in der deutlich besseren technischen Handhabbarkeit, da durch die Eintopfsynthese mehrere Aufarbeitungs- und Reinigungsschritte eingespart werden können. Zum anderen konnte die benötigte Menge an Säure von 15eq. auf 5eq. herabgesetzt werden, was besonders im Hinblick auf die technische Synthese von großem Vorteil ist. Dadurch wird die bei der Neutralisation anfallende Salzmenge stark reduziert.

In dem erfindungsgemäßen Verfahren zur Herstellung von 4,5-Dihydro-1,3-thiazolen der Formel (I) werden vorzugsweise äquimolare Mengen Trialkoxyalkan und Cyanid, vorzugsweise Trimethylsilylcyanid, unter Zugabe von katalytischen Mengen einer Lewis-Säure, vorzugsweise ZnCl₂, in einem Temperaturbereich von 40 bis 100°C, bevorzugt in einem Temperaturbereich von 55 bis 70°C, für 3 bis 20 Stunden, bevorzugt in einer Zeit von 12 bis 18 Stunden, erhitzt. Nach dem Abkühlen gibt man 1.0 bis 1.5 Äquivalente, bevorzugt 1.0 bis 1.2 Äquivalente, 1-Amino-2-alkanthiol (II) in einem organischen Lösungsmittel dazu. Als organisches Lösungsmittel werden vorzugsweise polare Lösungsmittel, z.B. Alkohole, verwendet. Die Reaktionsmischung wird dann auf 40 bis 100°C erwärmt, vorzugsweise liegt die Reaktionstemperatur bei 60 bis 80°C. Die Reaktionsdauer liegt zwischen 3 und 20 Stunden, bevorzugt zwischen 12 und 18 Stunden. Das Lösungsmittel wird vorzugsweise im Vakuum abdestilliert. Zu der verbleibenden Reaktionsmischung wird bei einer Temperatur von 10°C bis -10°C, bevorzugt von 0°C bis 5°C, zwischen 5 und 30 Äquivalente, bevorzugt 5 - 15 Äquivalente, besonders bevorzugt 5 - 7 Äquivalente, einer Säure, vorzugsweise konzentrierte Schwefelsäure, zugetropft. Nach 1 bis 5 Stunden, bevorzugt nach 1 bis 3 Stunden, Rühren bei der oben genannten Temperatur wird mit einer wässrigen Base, bevorzugt NaHCO₃ neutralisiert. Nach der Extraktion des 4,5-Dihydro-1,3-thiazol (I) in eine organische Phase, wobei als Lösungsmittel bevorzugt Dichlormethan oder organische Ether, z.B. Diethylether, verwendet werden, isoliert man in einer Ausbeute von ca. 40 % die gewünschten Verbindungen.

### Beispiel 1: 2-(1,1-Dimethoxypropyl)-4,5-dihydro-1,3-thiazol (IV) - Einzelsynthese

Unter Argon werden 20.04 g wasserfreies Ammoniumacetat (260 mmol), 6.79 g (88 mmol) Cysteamin und 10.33 g (80 mmol) 2,2-Dimethoxybutyronitril in 80 ml abs. Methanol gelöst und 16 h unter Rückfluss erhitzt. Nach dem Abdestillieren des Lösungsmittels im Vakuum wird die Reaktionslösung in Portionen zu einer Mischung von 18.4 g KOH, 164 ml Eiswasser und 40 ml Diethylether gegeben. Die Phasen werden getrennt und die wässrige Phase mit Diethylether (5 x 10 ml) extrahiert. Nach dem Trocknen der vereinigten organischen Phasen über NaSO₄ und KOH-Plätzchen wird die Lösung eingeengt und kann direkt zu 2-Propionyl-4,5-dihydro-1,3-thiazol umgesetzt werden.

Rohausbeute: 13.89 g (73.4 mmol, 91.7 %).
¹H-NMR (400MHz; CDCl₃): 0.85 (t, 3H, CH₃); 1.94 (q, 2H, CH₂); 2.27 (t, 8H, CH₂S und OCH₃); 4.38 (t, 2H, CH₂N)

### Beispiel 2: 2-Propionyl-4,5-dihydro-1,3-thiazol (I) - Einzelsynthese

Zu 43 ml Schwefelsäure (96 %) werden bei 0-5°C 10.13 g (53.5 mmol) 2-(1,1-Dimethoxypropyl)-thiazolin zugegeben. Nach 20 min. Rühren bei dieser Temperatur wird die Lösung in Portionen zu einer Mischung von 187 mg NaHCO₃, 965 mg Eis und 64 ml Diethylether gegeben. Nach der Phasentrennung, Extraktion der wässrigen Phase mit CH₂Cl₂ und Trocknen der vereinigten organischen Phasen über Na₂SO₄ wird das Lösungsmittel im Vakuum entfernt.

Der Rückstand wird über eine Vigreux-Kolonne destilliert und man erhält 3.099 g (21.6 mmol, 40 % Ausbeute) Produkt mit einer Reinheit von 98 % lt. GC. ¹H-NMR (400MHz; CDCl₃): 1.14 (t, 3H, CH₃); 2.95 (q, 2H, CH₂); 3.33 (t, 2H, CH₂S); 4.52 (t, 2H, CH₂N).

### Beispiel 3: Eintopfsynthese zu 2-Propionyl-4,5-dihydro-1,3-thiazol (I) - Erfindung

Unter Argon werden 536 mg 1,1,1-Trimethoxypropan (4 mmol), 0.53 ml Trimethylsilyl-cyanid (4 mmol) und 1 mg ZnCl₂ 16 h auf 60°C erhitzt. 339 mg Cysteamin (4.4 mmol), 154.2 mg Ammoniumacetat (2.0 mmol) und 4 ml Methanol werden dazugegeben und weitere 17 h am Rückfluss erhitzt. Nach dem Entfernen des Lösungsmittels im Vakuum werden bei 0-5°C 2.043 g Schwefelsäure (96 %) zugetropft. Nach 2 h Rühren bei dieser Temperatur wird die Reaktionslösung in Portionen zu einem Gemisch von 4.7 g NaHCO₃ (56 mmol), 75 ml Eiswasser und 5 ml Diethylether gegeben. Die wässrige Phase wird mit CH₂Cl₂ extrahiert, die vereinigten organischen Phasen über NaSO₄ getrocknet und im Vakuum eingeengt.
Ausbeute (Rohprodukt): 229 mg (40 %); Reinheit lt. GC: 84%.

## Patentansprüche

1. Verfahren zur Herstellung von 4,5-Dihydro-1,3-thiazolen der Formel (I) nach der nachfolgenden Reaktionssequenz 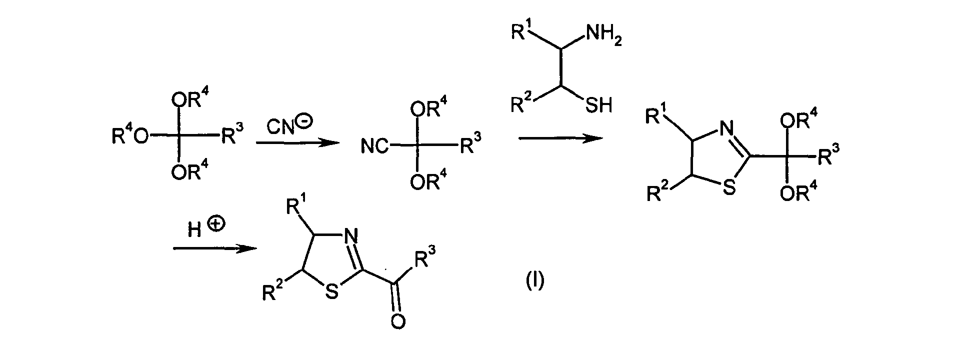 wobei R¹, R², R³ und R⁴ - unabhängig voneinander - Wasserstoff oder einen organischen Rest mit 1 bis 10 Kohlenstoffatomen bedeuten,
**dadurch gekennzeichnet, dass** die gesamte Reaktionssequenz als Eintopfsynthese ohne Isolierung der Zwischenstufen durchgeführt wird.

2. Verfahren nach Anspruch 1, **dadurch gekennzeichnet, dass** R³ Ethyl bedeutet.

3. Verfahren nach Anspruch 1, **dadurch gekennzeichnet, dass** CN^{⊖} Trimethylsilylcyanid bedeutet.
